# EUROPEAN PATENT SPECIFICATION

(11) **EP 2 030 612 B1**
(45) Date of publication and mention of the grant of the patent: **23.04.2014**
(21) Application number: 07794054.2
(22) Date of filing: 15.06.2007
(51) Int. Cl.: A61K 9/06, A61K 47/10, A61K 47/36, A61K 47/38, A61K 31/505, A61P 17/02, A61L 26/00, A61K 33/38

(54) **PHARMACEUTICAL COMPOSITION FOR TREATING BURNS AND A METHOD FOR THE PRODUCTION THEREOF**
PHARMAZEUTISCHE ZUSAMMENSETZUNG ZUR BEHANDLUNG VON VERBRENNUNGEN UND VERFAHREN ZU IHRER HERSTELLUNG
COMPOSITION PHARMACEUTIQUE POUR TRAITER DES BRÛLURES ET PROCÉDÉS DE FABRICATION

(30) Priority: 19.06.2006 RU 2006121794
(43) Date of publication of application: 04.03.2009
(73) Proprietor: Rasnetsov, Lev Davidovich, Nizhny Novgorod, 603000 (RU)
(72) Inventor: SHVARTSMAN, Iakov Yudelevich, Nizhny Novgorod, 603105 (RU); YASHNOVA, Olga Konstantinovna, Nizhny Novgorod, 603016 (RU); MELNIKOVA, Nina Borisovna, Nizhny Novgorod, 603074 (RU); SOROKIN, Pavel Vladimirovich, Nizhny Novgorod, 603146 (RU); ZIMNYAKOVA, Olga Evgenyevna, Nizhegorodskaya oblast, 606860 (RU); RASNETSOV, Lev Davidovich, Nizhny Novgorod, 603000 (RU)
(74) Representative: HOFFMANN EITLE
(86) International application number: PCT/RU2007/000327
(87) International publication number: WO 2008/002196

(56) References cited:
- RU-C1- 2 102 975
- RU-C1- 2 259 816
- DATABASE WPI Week 200062 Thomson Scientific, London, GB; AN 2000-645780 XP002691266, & RU 2 148 989 C1 (AS RUSSIA KAZAN ARBUZOV ORG & PHYS CHEM) 20 May 2000 (2000-05-20)
- DATABASE CA [Online] CHEMICAL ABSTRACTS SERVICE, COLUMBUS, OHIO, US; IZMAYLOV, S. G. ET AL: "Adhesive paste for prophylaxis and treatment of trophic ulcers", XP002691271, retrieved from STN Database accession no. 150:129150 & RU 2004 103013 A (INST ORCH I FIZICHESKOJ KHIM I [RU]) 10 July 2005 (2005-07-10)
- RUSLAN MASGUTOV ET AL: "Stimulation of the Rat's Sciatic Nerve Regeneration by Local Treatment with Xymedon(R)", CELLULAR AND MOLECULAR NEUROBIOLOGY, KLUWER ACADEMIC PUBLISHERS-PLENUM PUBLISHERS, NE, vol. 26, no. 7-8, 26 May 2006 (2006-05-26) , pages 1411-1419, XP019447875, ISSN: 1573-6830, DOI: 10.1007/S10571-006-9055-7
- IZMAILOV S.G. ET AL.: 'Ksimedon: nastoyasche i budusche' NMZH, [Online] 2002, page 1, 3 - 4, XP008101790 Retrieved from the Internet: <URL:http://www.medicum.nnov.ru/nmj/2002/3/ 18.php>
- AGROSULFAN: 'Spravoshnik Vidal' LEKARSTVENNYE PREPARATY V ROSSII, [Online] Retrieved from the Internet: <URL:http://www.vidal.ru/vidal/info/drugs/d 9570.htm>

## Description

### Technical Field

The invention relates to medicine, in particular to soft medicinal agents for external application (ointment, gels, emulsions, liniments and can be used for treating thermal, solar and chemical burns of human beings and animals.

### Prior Art

Burns (various degrees of severity) are accompanied by disturbances of an alkali-acid balance and the synthesis of proteins, the combination of fermentative reactions of disintegration of complex high-molecular and bioorganic compounds, a factor that results in tissue necrosis, exudate reactions, formation of granulation tissue, bacterial infection of a burn surface. The search for means excellent in treating burn wounds is a problem of current interest of modem medicine and pharmacology.

Known are various soft medicinal forms of wound-healing means by which to allow the treatment of burns.

For instance, known are apparatuses for treating burns on active substance - methyluracyl basis having regenerative activity. Ointment "Methyluracyl" for local and external application (cf. the Register of Medicinal of Russia") contains 10 g of methyluracyl and an ointment base (vaseline, medical; lanoline, aqueous) up to 100 g. Likewise, known is antimicrobial locally applicable ointment "Levomecol" containing 4 g of methyluracyl, 0.75 g of levomycetin and an ointment base up to 100 g. Despite the fact that methyluracyl ointments are currently the most widespread means for treating burns, they are not devoid of some defects. It is necessary to mention the defects of methyluracyl, such as absence of antimicrobial activity, which dictates the need for administration of antimicrobial components; on mixture with a wound secretion, there occurs inactivation of a preparation; on sterilization, the preparation loses its therapeutic activity and becomes a placebo. Besides, a vaseline-lanoline base impedes normal gas exchange, which impairs the wound-healing activity of methyluracyl.

Likewise, known are medicinal agents for treating burns in the form of gels, which are distinguished for employment of various active substances and gel-forming components.

For example, known is a compound for treating burns in children (cf. patent RF 2209074, published 2003) containing a bioactive component of preparation Actovegin, injectable formulation in the form of a solution and gel Tisol with the following ratio of components, wt%: said Actovegin solution - 0.2-0.3 as calculated in terms of the active component, Tisol - up to 100.

The compound for treating burns is prepared in the following manner. In a reactor provided with a heating system and a mechanical mixer is prepared a Tisol compound base - substance to add it with Actovegin in the form of an injectable solution in ampules based on the obtainable Actovegin concentration as determined - 0.2 to 0.3 wt%. The formulation so obtained is vigorously mixed up to a homogeneous medium, performing the quality control of the mixture and packaging in glass tare (dim glass) 10-15 g each, closing and pasting up labels.

The compound for treating burns in children is applied in the following manner.

After the first toilet of a burn wound comprising covering and evacuating the epidermal cysts, removing the residue of the affected epidermis, cleaning the wound with gauze antiseptic wetted tampons, the claimed compound is applied - Tisol with Actovegin. The defect of the compound are allergic responses in some patients (nettle rash, fever, temperature rise, shock), a factor that is particularly taken account of in the treatment of children.

Another example is a wound-healing agent (cf. patent RF No 2259816/ published 2005) including an active substance Mexidol (2-ethyl 6-methyl 3-oxypyridine succinate), a gel former and distilled water, with the following ratio of components, wt%: the gel former 0.5-5.0, a stabilizer 0.01-0.8, a preservative 0.01-1.0, mexidol 0.01-10.0, water, distilled, deionized - the balance. The wound-healing agent is prepared in the following manner. In a special reactor is placed distilled deionized water in amounts required, heating same to 70°C and adding a gel forming base and the stabilizer under constant stirring. Furthermore, a preservative is dissolved in a separate vessel under stirring at room temperature, by sequentially adding the mexidol to obtain the complete transparency of the solution. The solutions thus obtained are mixed under stirring for two hours up to the complete transparency and uniformity of a system. The obtainable gel is cooled to room temperature. The known agent is highly efficient, shows a well-pronounced wound-healing effect in 1, 2, 3 phases of a wound process, quickly eliminates inflammatory processes, diminishes painfulness, edema, performs particularly well with affected tissue condition, accelerates reparative-degenerative processes and epithelization terms and demonstrates a wide range of action. The known wound-healing agent is disadvantageous in that mexidol as an oxidant shows a therapeutic effect only in the initial period of application of a medicinal agent onto a wound surface. The more intensive the inflammatory processes, the more intensive the accumulation of products of lipids peroxide oxidation and, accordingly, mexidol is consumed more rapidly and more than that the main portion of the active substance is inactivated in an aqueous phase by means of atmospheric oxygen. Said processes are intensified owing to the fact that a monomolecular film is formed on a skin surface being substantially a membrane and boosting mexidol inactivation. Besides, mexidol exhibits instability in antiseptic conditions, more exactly, in the event of commercial package defect, the active substance is inactivated rapidly. In view of the foregoing, mexidol is a "first aid" preparation: its action is very efficient, albeit of short duration, which is not rational in the therapy of burns.

Also known is an antiburn agent "Xymedon" - N -(β-oxycthyl)-4, 6-dimethyldihydropyrimedon-2, pyrimidine derivative, 3 generation, having high regenerative activity (cf. I.C. No 1685454, published 1991). Xymedon is an effective agent for treating burns due to high anti-inflammatory and regenerative activity thereof. A therapeutic effect is expressed in the rapid healing of a wound surface, the falling-off of a scab in earlier periods, improvement of morphological and pure-chemical picture of skin damages and restoration of biochemical indices indicating metabolic processes to be normalized. On the basis of this agent a number of preparations have been created. For instance, known is a medicinal agent for treating burns (cf. "Xymedon in clinical practice", S.G. Izmailov et al., Nizhni Novgorod, NGMA Publishers, 2001, p. 92) in the form of two medicinal forms - ointment and powder, said ointment containing as an active agent, xymedon (5-10 wt%) and as base-forming agent a vaselino-lanoline mixture (lanoline - 40-45, vaseline-the balance (up to 100 wt%). The powder contains xymedon, (5-10 wt%) and talc - the balance (up to 100 wt%). All the medicinal forms as proposed are prepared according to universally adopted pharmacopoeia rules. The agent has antibacterial activity, reduces hydration and dehydration phases of a wound process and terms of wound treatment.

More, known is employment of xymedon in the form of an aqueous solution, ointment and powder which call for treating particular wounds and trophic ulcers for a remarkably long time (cf. patent RF No 2148989, published 2000).

Russian patent application No. 2004-103013 discloses a glutinous paste comprising xymedon, hydrocortisone, boric acid, zinc oxide, glycerin, gelatin and water for the treatment of trophic ulcers.

The Applicant is unaware of a composition on active substance "Xymedon" basis in the form of a gel and, as so, any one of the inventions as described on the basis of xymedon can be taken as prototype. Applicant's prototype selected is represented by a composition in the form of an ointment, as shown and described above (cf. Xymedon in clinical practice", op. cit.). The prototype for a method of producing a pharmaceutical composition, the Applicant has selected, is a method for producing a wound-healing agent (cf. patent RF 2259816, published 2005), as shown and described above.

### Essence of the Invention

The invention is directed to the solution of a task consisting in obtaining a highly effective regenerative wound-healing, micro-circulation improving means for treating infected burn wounds, executed in the form of a gel - medicinal form exerting the most favorable effects in the treatment of burns based on active substance "Xymedon"- (N-(β-oxyethyl)-4,6-dimethyldihydropyrimedon-2-)-pyrimidine derivative, 3 generation, having high regenerative activity.

(For the task set to be solved, claimed is a group of inventions combining the variants of a pharmaceutical composition for treating burns on the basis of an active substance "Xymedon", containing as base-forming means a gel former, a moisture retaining agent and distilled water and methods for producing compositions.

A pharmaceutical composition for treating burns includes an active substance (N-(β-oxyethyl)-4,6-dimethyldihydropyrimedon-2 (xymedon) and base-forming substances, in which, according to the invention, the base-forming substances contained therein are represented by a gel former representing sodium salts of biopolymers, a moisture retaining agent representing glycerin in an amount of not less than 20 wt %, a stabilizer, a preservative and distilled water. It can contain, as the gel former, sodium carboxymethylcellulose, sodium alginates or a mixture thereof.

On use of sodium carboxymethylcellulose, as a gel former, the composition has the following makeup, wt%:

| | |
|---|---|
| Xymédon | 1.0-10.0 |
| Sodium carboxymethyl cellulose | 2.0-3.0 |
| Glycerin | 20.0-30.0 |
| Stabilizer | 0.4-1.0 |
| Preservative | 1.0-2.0 |
| Distilled water - | balance up to 100 |

On use of sodium alginates, as a gel former, the composition has the following makeup, wt %:

| | |
|---|---|
| Xymedon | 1.0-10.0 |
| Sodium alginates | 4.0-6.0 |
| Glycerin | 20.0-30.0 |
| Stabilizer | 0.4-1.0 |
| Preservative | 1.0-2.0 |
| Distilled water - | balance up to 100 |

On use of a mixture of sodium carboxymethylcellulose and sodium alginates, as a gel former, the composition has the following makeup, wt%:

| | |
|---|---|
| Xymedon | 1.0-10.0 |
| Sodium carboxymethyl | |
| cellulose | 1.0-3.0 |
| Sodium alginates | 0.2-0.4 |
| Glycerin | 20.0-30.0 |
| Stabilizer | 0.4-1.0 |
| Preservative | 1.0-2.0 |
| Distilled water - | balance up to 100. |

A pharmaceutical composition for treating burns, according to a second alternative embodiment of the invention, in addition to an active substance "Xymedon" contains an active substance-silver nitrate in an amount of 0.1-0.2wt%.

A pharmaceutical composition for treating burns, according to a third alternative embodiment of the invention, in addition to an active substance "Xymedon" contains active substances - silver nitrates in an amount of 0.1-0.2 wt% and sodium sulfacyl in an amount of 0.5-1.0 wt%.

A pharmaceutical composition for treating burns, according to a fourth alternative embodiment of the invention, in addition to an active substance "Xymedon" contains active substances-levomycetin (1.0-5.0 wt%) and succinic acid (2.0-10.0 wt%).

Likewise, according to the invention, said technical result is attained by the variants of a method for preparing a pharmaceutical composition for treating burns. A first variant allows one to obtain the first three variants of pharmaceutical compositions and consists in that in a reactor with a water jacket in a mixture of distilled water and a moisture retaining agent, heated to a temperature comprised between 50 and 60°C a gel former is dissolved under stirring to form a uniform homogeneous mass whereupon the reactor is added with a preservative and a stabilizer, a moiety of the base thus obtained being added to the calculated quantity of one or several active substances being first comminuted in a colloidal mill, mixing same up to the uniform mass being returned thereafter to the reactor to mix with the remaining part of the base before the uniform mass has been formed.

A second embodiment of a method allows one to obtain the fourth embodiment of a pharmaceutical composition for treating burns and consists in that while additionally including in any one of four compositions, of active substances-levomycetin in an amount of 1.0-5.0 wt% and succinic acid - 2.0-10.0 wt%, these are dissolved in water heated to 70°C in another reactor with a water jacket by sequentially mixing the solution so obtained with a moiety of a base containing xymedon.

The essence of the claimed invention consists in that on the basis of the known burn treatment agent --- xymedon there have been created a number of compositions in the form of a new medicinal form - gel, a most favorable form for treating burns. The technical result in the present invention is achieved by not only using a basic active substance-xymedon but also synergism and a sustained release of its components.

Xymedon is an organic base as to a chemical nature thereof. In aqueous media there form more hydrophilic and ionic structures owing to lactim-lactam tautomerism, which are capable of radically changing not only interaction of xymedon with the other components of a pharmaceutical composition but also affecting a process of wound-healing the different ways.

A change in the hydrophilic-lipophilic ratio in the molecule of xymedon occurs under the action of pH media, presence of other active substances or metal ions.

Of substantive importance are the adsorption properties of xymedon relative to a burn wound surface from aqueous media, inasmuch as these determine penetrability of substances in the damaged skin parts. Both the time of xymedon adsorption and a concentration of the most active xymedon form are essential.

One of the methods of a more effective delivery of a medicinal agent-xymedon is its capture by a structured net of ionized biopolymers in saline form (sodium salts). During xymedon sorption on polymer chains containing Na⁺ ions there originate more reactive ionized xymedon forms in the form of sodium salts having higher penetrability to wound tissues. Adhesion of the biopolymers with xymedon attached complexes to a burn wound provides the sustained action of an active substance as well.

To achieve these effects, a gel former proposed in the invention is represented by sodium salts of biopolymers -carboxymethylcellulose (Na-CMC) and alginic acid (sodium alginate). Na-CMC and sodium alginates are known as biocompatible materials of natural polysaccharides having resolution effects and precluding exudation. The basis of the capability of these materials of being resolved rests in their solubility in water and plasma. They are hydrophilic, exhibit high adsorption capability (up to 5000 %), are responsible for good adhesion to wound, show no toxicity and irritant action and demonstrate good hemostatic properties.

Alginates contribute to stimulation of granulation processes in tissues and epithelization thereof.

However, the use of Na-CMC and Na alginate as biocompatible water base-free coverages do not provide a good mechanical strength of the coverage and stability on a burn wound (S.G. Shapovalov. Sovremennye ranevye pokrytiya v kombustologii. "FARMindexPraktik" issue 8, 2005, pp. 38-46).

On their introduction into a water-or water-glycerin base, Na-CMC and Na alginate perform the following functions:
- draining sorbents. These substances provide the drain of not only an exudate in a bandage in a burn wound but also micro-organisms;
- a matrix for the delivery of medicinal agents to wound thereby to reduce a dosage of said agents scores of times;
- a gel former and a cross-linking component.

According to the present-day notions, a gel is formed due to the formation of zones of fusion, more exactly, connective zones between the segments of a polymer chain with regularly repeating structures under conditions facilitating such an association of chains (Pilnic W, Rombouts F Polysaccharides and food processing //Carbohydr. Res. 1985. V 142. P. 93-105).

In alginate gels, the adjacent polysaccharide chains form zones of fusion on account of hydrogen bridges between the carboxylic groups and association of the chains occurs in consequence of the formation of multiple bonds with positive ions or polar medicinal substances of small size which are positioned between the molecules of alginate like eggs in a package ("egg-box structure" (Yu.S. Khotimchenko, V.V. Kovalev, O.V. Savchenko, O.A. Ziganshina. Physical-chemical properties, physiological activity and application of alginates - polysaccharides of brown algae. Biologiya morya. 2001, vol. 27, No 3, pp. 151-162)). Besides, the use of a mixture of sodium salts of biopolymers (Na-CMC and Na alginates) increases a sustained release of xymedon due to the formation of a cellular structure of polymer nets capable of firmly capturing the molecules or ion xymedon complexes. The jointly formed structure permits, at a lesser concentration of polymers, providing a composition viscosity as required and is a trap for micro-organisms.

A gel former is present in a composition in effective amounts. An amount below a lower limit does not provide a sufficient viscosity of the composition, which fact does not allow one to fix it on a burn wound; an amount above an upper limit causes technological difficulties in the manufacture of the composition, namely mixing of a solution is made difficult as is packing of a finished product.

The sustained release of a composition is likewise enhanced when glycerin is used as a moisture retaining agent, the use of which is responsible for mitigating burn tissues, their swelling, which fact intensifies xymedon penetrability thru wound tissues. Said glycerin, unlike a majority of moisture retaining agents, say, a urea, glycols, is a chemically indifferent substance relative to xymedon and is present in the composition in an amount of 20.0-30.0 wt%. With its content being less than 20.0 wt%, the ability of a formulation to retain moisture is reduced. With a content of more than 30 wt%, a moisture retaining effect does not increase.

The stabilizer and preservative used are represented by substances traditional in the pharmacopoeia industry. For example, the former is Nipagin, the latter - potassium sorbate or sorbic acid. Said sorbic acid performs particularly well with subacid media (pH ≤ 6.5) and said potassium sorbate, in neutral and weak alkali media.

The second embodiment of a pharmaceutical composition in addition to xymedon comprises an active substance-silver nitrate which is known as a bactericidal, anticeptic, viscous and caustic substance (The State Register of Medicinals). In the polymer matrix of polysaccharides, such as Na-CMC, Na alginates or a mixture thereof in aqueous media, silver ions are reduced to O-valency state (colloidal silver) stabilized in the matrix. The formation of oxidized forms of polysaccharides at the time of reduction of silver ions promotes a further progress of their reduction.

In presence of xymedon, silver nitrate forms insoluble precipitates. These compounds in a polymer matrix, in the presence of oxidized forms of polysaccharides and starting polysaccharides themselves likewise form the clusters of silver nanoparticles. This being so, the introduction of said silver nitrate into a composition with xymedon results in appreciably changing the pharmacological properties of a gel. Colloidal silver nanoparticles show a much wider range of action, specifically, apart from known bactericidal properties, they are used as immunomodulators, and demonstrate an antiviral effect against a vaccinia virus (Cf. Bogdanchikova N.E., Kourbatov A.V., Tretiyakov V.V., Rodionov P.P. "Activity of colloidal silver preparations against vaccinia virus". Chemico-pharmaceutical journal, 1992, No 9-10, p. 90; Derzhavin A.E., Turchanikov R.O., Ozaryanskaya N.M. "Problems and Perspectives of Clinical Use of Silver Preparations (literature review). Vrachebnoye Delo, 1987, No. 8, pp. 114-119) et al.

The combination of xymedon with silver ions, silver ions in a polymer matrix of biopolymers, glycerin in a gel contributes to intensifying burn wound-healing.

The third embodiment of a pharmaceutical composition further comprises silver nitrate and sulfacyl sodium. The introduction into a pharmaceutical composition containing xymedon, a gel former (Na-CMC, sodium alginates or mixtures of said biopolymers), glycerin, water, preservatives and stabilizers, silver nitrate and a water-soluble preparation -sulfacyl sodium from the group of sulfanilamide preparations enables not only to widen a range of antimicrobial action but also to necessitate the new qualities of the composition.

N-aminobenzenesulfonylacetamide sodium hydrate (Sulafacyl sodium) in aqueous media on a wound surface forms a protective monomolecular film of sulfacyl silver.

Said sulfacyl silver has low solubility, does not react with xymedon, glycerin and other components of a composition to form new substances.

A medicinal agent is delivered to a burn wound in a matrix of polymers. Sulfacyl silver is captured either by a three-dimensional Na-CMC net structure or else the agent gets into the cells of the three-dimensional structure of Na alginates.

Known are silver salts of sulfamide preparations, such as a sulfazin silver salt, Argosulfane, sulfargin, which are used for treating burn wounds.

The preparations represent ointments and creams:
1. 1% ointment - sulfazin silver salt - ointment "Sulfargin" for treating purulent wounds and bums;
2. Cream "Dermazin" - 1% sulfazin silver salt;
3. Cream "Argosulfane" - 0.02% sulfathiazole silver; formulation: sulfathiazole silver salt - 20 mg, a base - up to 1 g (liquid paraffin, cetostearyl alcohol, vaseline, sodium laurylsulfate, glycerin, preservatives, water).

Preparations of sulfanylamide silver salts are antibacterial locally applicable preparations contributing to wound-healing from infection, relieve pain and burning in a wound, reduce the time of treatment and preparation of the wound for skin transplantation, improve conditions in humans in many cases to preclude the need of transplantation. (The Register of Medicinals of Russia - encyclopaedia of drugs". The 7th edition, revised and enlarged, 2000; M.D. Mashkovsky "Medicinal Agents", Two parts, Part II - the 12h edition, revised and enlarged - M.: MEDITSINA Publishers, 1994. - 688 pages).

Preparations have a wide range of bacteriostatic action relative to Gram positive and Gram negative bacteria. A mechanism of the antimicrobial action of sulfanylamide preparations - growth suppression and multiplication of microbes - is associated with a competitive antagonism with para-aminobenzoic acid (PABA) and dihydropteroate synthetase suppression, which is likely to disturb the synthesis of dihydrofolic acid and eventually its active metabolite - tetrahydrofolic acid required for the synthesis of purines and pyrimidines of a microbe cell. Silver ions present in the preparation strengthen several scores of times the antimicrobial effect of sulfanylamide - these inhibit the growth and division of bacteria by way of binding to microbe cell DNA. Besides, the silver ions are weakening the sentisizing properties of sulfanylamide.

Sulfacyl sodium in the presence of silver nitrate likewise has the entire complex of useful properties, as mentioned hereinabove. In combination with xymedon having another mechanism of effect on an organism (i.e. pharmacologically compatible and non-competitive), the components of an aqueous pharmaceutical composition (Na - CMC, Na alginate, glycerin, preservatives), the sulfacyl sodium and silver ions demonstrate excellent wound-healing, immunomodulation and reduce the sentisizing properties of sulfanylamides. Besides, the merit of using the pharmaceutical composition including, as active substances, xymedon, sulfacyl sodium and silver nitrate is high time stability of an aqueous gel form and considerable bioavailability of all the components of the composition.

The fourth embodiment of a pharmaceutical composition further contains active substances - levomycetin and succinic acid. The introduction into the pharmaceutical composition containing xymedon, a gel former (Na-CMC, Na alginates or mixtures of said biopolymers), glycerin, water, preservatives and stabilizers, levomycetin and succinic acid allows not only to widen the spectrum of antimicrobial action but also necessitates the new properties of the composition.

Levomycetin shows antimicrobial, antibacterial (bacteriostatic) action. It is efficient in relation to many Gram positive and Gram negative bacteria. Succinic acid produces positive effects on oxygenation processes, stabilizes the structure and functional activity of mitochondria, is an inductor of synthesis of certain proteins and exerts an influence on ion exchange in a cell. Said succinic acid impedes inflammatory processes (normalizes the contained histamine and serotonin), enhances microcirculation in organs and tissues and increases the effect of other drugs. Application of said succinic acid with other preparations enables to obtain a more well - pronounced therapeutic effect.

Thus, the introduction of levomycetin and succinic acid in addition to an active substance "Xymedon" allows one to obtain a highly effective regenerating, wound-healing, microcirculation improving agent in the form of a gel for the treatment of an infected burn wound.

Active substances - xymedon, silver nitrate, sulfacyl sodium, levomycetin and succinic acid are present in pharmaceutical compositions in effective amounts. An amount below the one that has been indicated produces no effect while an amount above the one that has been indicated is not desired because no increment is seen in the effect.

### Best Modes of Carrying out the Invention.

Table 1. The examples show the formulations of variants of pharmaceutical compositions as claimed for treatment of burns. The number of ingredients are given on a 100 wt% composition. The stabilizer used in all the examples is Nipagin in an amount of from 0.4 to 1.0 for 100 g of the composition.

Example I. Composition makeup, wt%:
Xymedon - 1,0; silver nitrate - 0.13; glycerin - 20.0; sodium carboxymethylcellulose - 2.0; stabilizer (Nipagin) - 0.4; preservative (potassium sorbate) - 2.0; distilled water - the balance up to 100.

Method of production of composition. In a reactor with a water jacket in a mixture of distilled water and glycerin, heated to a temperature comprised between 50 and 60°C, is dissolved a gel former under stirring - sodium carboxymethylcellulose to form a homogeneous mass whereupon said reactor is added with a preservative - potassium sorbate and stabilizer - Nipagin, adding a moiety of the base so obtained to the calculated quantity of one or several active substances - xymedon and silver nitrate previously comminuted in a colloidal mill and mixed to a homogeneous mass by sequentially returning same to the reactor and mixing with the remaining portion of the base to obtain the homogeneous mass.

Examples 2-4, 7-10, 12-14 are carried out in accordance with the method, as shown and described in Example I. For a composition makeup see Table 1.

Example 5. Composition makeup, wt%:
Xymedon - 5,0; levomycetin - 1.0; succinic acid - 2.7; glycerin - 30.0; sodium carboxymethyl cellulose - 2.0; stabilizer (Nipagin) - 1.0; preservative (sorbic acid) - 0.2; distilled water - the balance up to 100.

Method of preparation of composition. In a reactor with a water jacket in a mixture of distilled water and glycerin, heated to a temperature of from 50 to 60°, a gel former - sodium carboxymethylcellulose is dissolved to form a uniform homogeneous mass whereupon a preservative (sorbic acid) is introduced into the reactor along with a stabilizer (Nipagin), simultaneously dissolving in a second reactor with a water jacket in water heated to 70°C, active substances - levomycetin and succinic acid, adding a moiety of the base from the first reactor and the solution so obtained from the second reactor to the calculated quantity of xymedon, previously comminuted in a colloidal mill, and mixing same to a uniform mass which is then introduced into the first reactor and mixed with the remaining portion of the base to form the uniform mass.

Examples 6, 11, 15, 16 are realized in accordance with the variant of a method, as called for in Example 5. For a composition makeup see Table 1.

Samples of pharmaceutical compositions - gels with xymedon were submitted for pre-clinical examination. The efficacy of pharmaceutical compositions, externally applicable, was studied experimentally for 10 days on male Wistar rats as compared to a preparation - methyluracyl ointment (10%).

In the experiment, use was made of 48 mongrel rats (males, a body weight 220-250 g) divided into 8 groups, 6 animals each:
1 - Control I burn wound
2 - Control II placebo (vaseline oil)
3 - Burn wound + treatment with preparations
   ^{x} Group I- gels Nos. 7, 9, 12
   ^{x} Group 2 - gels Nos 1, 2, 3, 8, 13
   ^{x} Group 3 - gels Nos 4, 10, 14
   ^{x} Group 4 - gels Nos 5, 6, 11, 15, 16
4. Burn wound + treatment with a comparison preparation (methyluracyl ointment (10%)).

Test substances (as medicinal agents) were used in a dose of 10 mg/cm². The control substance used was represented by medical vaseline oil.

The efficacy of compositions was studied on models of an infected Staphylococcus aureus burn skin wound, IIIb degree, an area of 23.5 cm² with rats (7% of the total surface of a body). Modeling of a burn wound calls for recording the general condition of animals, rate of healing of a wound surface (Table 2), number of colony forming units (COE) in the wash-off from the burn wound.

The degree of variations of a burn wound in animals was similar upon the whole. IIIB degree burns are usually accompanied by the formation of a thick dense, primary burn scab, brown hue.

By the end of day 3 of treatment already, the scab of the animals in experimental groups was rejected along the edges of a wound surface, with a slight epithelization of wound edges observed, as distinct from the control animals, where such dynamics was not observed. In the group with treatment with methyluracyl ointment, all the animals showed an earlier rejection of scab crusts, the formation of wound exudate.

By the end of days 4-5 the positive dynamics of regenerative processes was observed in the animals of experimental groups which received treatment with gels with xymedon: the slow rejection of a scab, under which the regenerative processes proceeded actively, the well pronounced edge epithelization of a burn wound. In the group with treatment with methyluracyl ointment, in all the animals, in places of scab crusts there formed deep wounds filled with granulation tissues, with a gradual epithelization along the edges thereof. In the control group, the animals started rejecting a dry scab along the edges of the burn wound, the latter was constricted, and a new epithelium tissue appeared gradually.

By the end of day 6 all the groups with treatment with the proposed samples of gels showed a similar picture of the condition of burn wounds. A burn scab had been rejected. The scab in the treatment groups was dry, thin, with active regenerative processes proceeding underneath. An edge epithelization had been intensified without the development of a concentric constriction of wounds.

The positive dynamics of treatment with gel compositions was also traced in the following 7-10 days of an experiment. The animals were active, the daily procedures of gel application did not disturb them, under a transparent layer of gel one could watch how under a mildly receding scab there actively develops a granulation tissue, there proceeds a process of full-value edge epithelization with no concentric constriction and rumen formation. Unlike the experimental groups, scab rejection in the control animals proceeded gradually (all 10 experimental days), reparative processes under its protection were slow along wound edges at risk of the development of concentric constriction and rumens. In the groups of animals receiving treatment with methyluracyl ointment, the reparative processes were more rapid than in the animals of the control group, but an earlier rejection of scab crusts caused the formation of deeper difficultly healing wounds (even by the end of day 10 of treatment), which probably resulted from the development of infection complications of which risk is present all the time.

According to microbiological investigations, on use of the samples of preparations with xymedon, a significant reduction of the infection of a material with aurous staphylococcus was observed in a wound.

Investigations go to show that all of the gels as proposed exhibit well-pronounced bactericidal activity relative to Staphylococcus aureus, expressed by a wound-healing effect in the initial phase of a wound process, and facilitate the earlier terms of a complete healing of burn wounds as against a preparation - methyluracyl ointment (10%).

The pharmaceutical composition so obtained in the present invention in the form of a gel meets all the medico-biological requirements imposed on creation of modem medicinal agents for treating burns and wounds: the gel is clear, which fact allows for watching wounds; contains medicinal substances having antibacterial and reparative properties; elasticity enables it to be used for complex-shaped surfaces; provides the most favorable microenvironment for wound-healing; high adsorption capability relative to wound exudate; prevents penetration of micro-organisms; sufficient gas (oxygen, carbonic acid) permeability required for active reparative processes; lacks local irritant and allergic effects; sterilization resistant; easily removable from a skin surface; convenient in use for medical personnel and patients.

It is hence only logical to see that the pharmaceutical composition of the present invention, in the form of a gel, can be used as highly efficient regenerative wound-healing, micro-circulation improving means for treating infected burn wounds, bedsores, erosions, trophic ulcers and other skin damages and mucous membranes.

**Table 1**

| Example | Xymedon, g | Silver nitrate, g | Sulfacyl sodium, g | Levomycetin, g | Preservatives, 9 | Succinic acid, g | Aqueous hydrophilic base (Glycerin 20-30 g) | |
|---|---|---|---|---|---|---|---|---|
| | | | | | | | Na-CMC g | Na-alginates, g |
| 1 | 1,0 | 0,13 | - | - | Potassium sorbate 2,0 | - | 2,0 | - |
| 2 | 5,0 | 0,13 | - | - | | - | 3,0 | - |
| 3 | 10,0 | 1,5 | - | - | | - | 2,0 | - |
| 4 | 5,0 | 0.13 | 1,0 | - | | - | 2,0 | - |
| 5 | 5,0 | - | - | 1,0 | Sorbic acid 0,2 | 2,7 | 2,0 | - |
| 6 | 10,0 | - | - | 5,0 | | 10,0 | 2,0 | - |
| 7 | 5,0 | - | - | - | Potassium sorbate 2,0 | - | 2,0 | - |
| 8 | 10,0 | 0,1 | - | - | | - | - | 5,0 |
| 9 | 5,0 | - | - | - | | - | - | 5,0 |
| 10 | 5,0 | 0,13 | 0,5 | - | | - | - | 6,0 |
| 11 | 5,0 | - | - | 1,0 | Sorbic acid 0,2 | 5,4 | - | 4,0 |
| 12 | 5,0 | - | - | - | Potassium sorbate 2,0 | - | 2,0 | 0,4 |
| 13 | 10,0 | 0,2 | - | - | | - | 1,0 | 0,2 |
| 14 | 5,0 | 0,13 | 0,5 | - | | - | 1,0 | 0,2 |
| 15 | 5,0 | - | - | 0,5 | Sorbic acid 0,2 | 1,5 | 2,0 | 0,2 |
| 16 | 5,0 | - | - | 0,1 | | 0,27 | 2,0 | 0,2 |

**Table 2**

| Time of treatment (days) | 3 | | 5 | | 6 | | 7 | | 10 | |
|---|---|---|---|---|---|---|---|---|---|---|
| Groups | scab | epith | scab | epith | scab | epith | scab | epith | scab | epith |
| Control I | ++++ | - | ++++ | + | ++++ | + | +++ | + | ++ | +++ |
| Control II | ++++ | - | ++++ | + | +++ | + | +++ | + | + | ++ |
| (vas. oil) | | | | | | | | | | |
| Group I | +++ | + | +++ | ++ | ++ | ++ | + | +++ | - | ++++ |
| Gels No 7, 9, 12 | | | | | | | | | | |
| Group 2 | +++ | + | +++ | ++ | ++ | ++ | ++ | ++ | + | +++ |
| Gels No 1, 2, 3, 8, 13 | | | | | | | | | | |
| Group 3 | +++ | + | ++ | ++ | ++ | ++ | ++ | +++ | ++ | +++ |
| Gels No 4, 10, 14 | | | | | | | | | | |
| Group 4 | +++ | + | +++ | + | ++ | ++ | ++ | +++ | + | ++++ |
| Gels No 5, 6, 11, 15, 16 | | | | | | | | | | |
| Methyluracil ointment, 10% | + | + | - | + | - | ++ | - | +++ | - | +++ |

| | | | | | | | | | | |
|---|---|---|---|---|---|---|---|---|---|---|
| Note: scab - scab rejection characteristics, epith - score of degree of epithalization | | | | | | | | | | |

## Claims

1. A pharmaceutical composition for treating burns comprising an active substance N-(β-oxyethyl)-4,6-dimethyldihydropyrimidon-2 (xymedon) and base-forming means, **characterized in that** the base-forming means contained therein is represented by a gel former representing the sodium salts of biopolymers, a moisture retaining agent-glycerin in an amount of no less than 20wt%, a stabilizer, a preservative, and distilled water.

2. The composition according to claim 1, **characterized in that** the gel former contained therein is represented by sodium carboxymethylcellulose, with the following ratio of components, wt%:
| | |
|---|---|
| Xymedon | 1.0-10.0 |
| Sodium carboxymethyl cellulose | 2.0-3.0 |
| Glycerin | 20.0-30.0 |
| Stabilizer | 0.4-1.0 |
| Preservative | 1.0-2.0 |
| Water, distilled | the balance up to 100 |

3. The composition according to claim 1, **characterized in that** the gel former contained therein is represented by sodium alginates, with the following ratio of components, wt%:
| | |
|---|---|
| Xymedon | 1.0-10.0 |
| Sodium alginates | 4.0-6.0 |
| Glycerin | 20.0-30.0 |
| Stabilizer | 0.4-1.0 |
| Preservative | 1.0-2.0 |
| Water, distilled | the balance up to 100 |

4. The composition according to claim 1, **characterized in that** the gel former contained therein is represented by a mixture of sodium carboxymethylcellulose and sodium alginates, with the following ratio of components, wt%:
| | |
|---|---|
| Xymedon | 1.0-10.0 |
| Sodium carboxymethyl cellulose | 1.0-3.0 |
| Sodium alginates | 0.2-0.4 |
| Glycerin | 20.0-30.0 |
| Stabilizer | 0.4-1.0 |
| Preservative | 1.0-2.0 |
| Water, distilled | the balance up to 100 |

5. The composition according to any one of claims 1-4, **characterized in that** it further comprises an active substance-silver nitrate in an amount of from 0.1 to 0.2wt%.

6. The composition according to any one of claims 1-4, **characterized in that** it further comprises active substances-silver nitrate, 0.1-0.2wt%, and sulfacyl sodium, 0.5 to 1.0wt%.

7. The composition according to any one of claims 1-4, **characterized in that** it further comprises active substances-levomycetin, 1.0-5.0wt%, and succinic acid, 2.0-10.0wt%.

8. A method for producing a pharmaceutical composition for treating burns according to any one of claims 1-6, **characterized in that** in a reactor with a water jacket in a mixture of distilled water and a moisture retaining agent, heated to 50-60°C, the gel former is dissolved under stirring to form a uniform homogeneous mass whereupon the reactor is added with a preservative and a stabilizer, adding a moiety of the base thus obtained to the calculated quantity of one or more active substances, previously comminuted in a colloidal mill, and mixing the matter to obtain the homogeneous mass which is then returned to the reactor and mixed with the remaining portion of the base to form the homogeneous mass.

9. The method according to claim 8, **characterized in that** while additionally including in the composition under any one of claims 1-4, active substances-levomycetin in an amount of from 1.0 to 5.0wt% and succinic acid in an amount of from 2.0 to 10.0wt%, they are dissolved in water heated to 70°C in another reactor with a water jacket, and the solution thus obtained is then combined with a moiety of the xymedon-containing base.

10. The composition according to any one of claims 1 to 7 for use in a method for treating burns.

## Patentansprüche

1. Pharmazeutische Zusammensetzung zur Behandlung von Verbrennungen, die den Wirkstoff N-(β-Oxyethyl)-4,6-dimethyldihydropyrimidon-2. (Xymedon) und basisbildende Mittel umfasst, **dadurch gekennzeichnet, dass** die darin enthaltenen basisbildenden Mittel durch einen Gelbildner, der Natriumsalze von Biopolymeren darstellt, ein Glycerin-Feuchthaltemittel in einer Menge von nicht weniger als 20 Gew.%, einen Stabilisator, ein Konservierungsmittel und destilliertes Wasser vertreten sind.

2. Zusammensetzung gemäß Anspruch 1, **dadurch gekennzeichnet, dass** der darin enthaltene Gelbildner durch Natriumcarboxymethylcellulose vertreten ist, mit dem folgenden Verhältnis der Bestandteile, Gew.%:
| | |
|---|---|
| Xymedon | 1,0 - 10,0 |
| Natriumcarboxymethylcellulose | 2,0 - 3,0 |
| Glycerin | 20,0 - 30,0 |
| Stabilisator | 0,4 - 1,0 |
| Konservierungsmittel | 1,0 - 2,0 |
| destilliertes Wasser | der Rest bis zu 100 |

3. Zusammensetzung gemäß Anspruch 1, **dadurch gekennzeichnet, dass** der darin enthaltene Gelbildner durch Natriumalginate vertreten ist, mit dem folgenden Verhältnis der Bestandteile, Gew.%:
| | |
|---|---|
| Xymedon | 1,0 - 10,0 |
| Natriumalginate | 4,0 - 6,0 |
| Glycerin | 20,0 - 30,0 |
| Stabilisator | 0,4 - 1,0 |
| Konservierungsmittel | 1,0 - 2,0 |
| destilliertes Wasser | der Rest bis zu 100 |

4. Zusammensetzung gemäß Anspruch 1, **dadurch gekennzeichnet, dass** der darin enthaltene Gelbildner durch eine Mischung aus Natriumcarboxymethylcellulose und Natriumalginaten vertreten ist, mit dem folgenden Verhältnis der Bestandteile, Gew.%:
| | |
|---|---|
| Xymedon | 1,0 - 10,0 |
| Natriumcarboxymethylcellulose | 1,0 - 3,0 |
| Natriumalginate | 0,2 - 0,4 |
| Glycerin | 20,0 - 30,0 |
| Stabilisator | 0,4 - 1,0 |
| Konservierungsmittel | 1,0 - 2,0 |
| destilliertes Wasser | der Rest bis zu 100 |

5. Zusammensetzung gemäß einem der Ansprüche 1 bis 4, **dadurch gekennzeichnet, dass** sie ferner einen Silbernitratwirkstoff in einer Menge von 0,1 bis 0,2 Gew.% umfasst.

6. Zusammensetzung gemäß einem der Ansprüche 1 bis 4, **dadurch gekennzeichnet, dass** sie ferner die Wirkstoffe Silbernitrat, 0,1 bis 0,2 Gew.%, und Sulfacylnatrium, 0,5 bis 1,0 Gew.%, umfasst.

7. Zusammensetzung gemäß einem der Ansprüche 1 bis 4, **dadurch gekennzeichnet, dass** sie ferner die Wirkstoffe Levomycetin, 1,0 bis 5,0 Gew.%, und Bernsteinsäure, 2,0 bis 10,0 Gew.%, umfasst.

8. Verfahren zur Herstellung einer pharmazeutischen Zusammensetzung zur Behandlung von Verbrennungen gemäß einem der Ansprüche 1 bis 6, **dadurch gekennzeichnet, dass** in einem Reaktor mit Wassermantel in einem auf 50 bis 60°C erwärmten Gemisch aus destilliertem Wasser und einem Feuchthaltemittel der Gelbildner unter Rühren gelöst wird, um eine gleichförmige homogene Masse zu bilden, woraufhin dem Reaktor ein Konservierungsmittel und ein Stabilisator zugesetzt werden, ein Teil der so erhaltenen Basis zu der berechneten Menge eines oder mehrerer Wirkstoffe, die zuvor in einer Kolloidmühle zerkleinert wurden, gegeben wird und die Masse gemischt wird, um eine homogene Masse zu erhalten, die dann in den Reaktor zurückgegeben und mit dem verbliebenen Teil der Basis gemischt wird, um eine homogene Masse zu bilden.

9. Verfahren gemäß Anspruch 8, **dadurch gekennzeichnet, dass** während der zusätzlichen Einarbeitung der Wirkstoffe Levomycetin in einer Menge von 1,0 bis 5,0 Gew.% und Bernsteinsäure in einer Menge von 2,0 bis 10,0 Gew.% in die Zusammensetzung gemäß einem der Ansprüche 1 bis 4, die Wirkstoffe in auf 70°C erwärmtem Wasser in einem anderen Reaktor mit Wassermantel gelöst werden und die so erhaltene Lösung dann mit einem Teil der Xymedonhaltigen Basis kombiniert wird.

10. Zusammensetzung gemäß einem der Ansprüche 1 bis 7 zur Verwendung in einem Verfahren zur Behandlung von Verbrennungen.

## Revendications

1. Composition pharmaceutique pour traiter des brûlures comprenant une substance active N-(β-oxyéthyl)-4,6-diméthyldihydropyrimidon-2 (xymédon) et un moyen de formation de base, **caractérisée en ce que** le moyen de formation de base contenu en son sein est représenté par un gélifiant représentant les sels de sodium de biopolymères, une glycérine comme agent de rétention d'humidité en une quantité non inférieure à 20 % en poids, un stabilisant, un conservateur et de l'eau distillée.

2. Composition selon la revendication 1, **caractérisée en ce que** le gélifiant contenu en son sein est représenté par de la carboxyméthylcellulose sodique, avec le rapport suivant de composants, en % en poids :
| | |
|---|---|
| Xymédon | 1,0 à 10,0 |
| Carboxyméthyl-cellulose sodique | 2,0 à 3,0 |
| Glycérine | 20,0 à 30,0 |
| Stabilisant | 0,4 à 1,0 |
| Conservateur | 1,0 à 2,0 |
| Eau, distillée | quantité suffisante jusqu'à 100. |

3. Composition selon la revendication 1, **caractérisée en ce que** le gélifiant contenu en son sein est représenté par des alginates de sodium, avec le rapport suivant de composants, en % en poids :
| | |
|---|---|
| Xymédon | 1,0 à 10,0 |
| Alginates de sodium | 4,0 à 6,0 |
| Glycérine | 20,0 à 30,0 |
| Stabilisant | 0,4 à 1,0 |
| Conservateur | 1,0 à 2,0 |
| Eau, distillée | quantité suffisante jusqu'à 100. |

4. Composition selon la revendication 1, **caractérisée en ce que** le gélifiant contenu en son sein est représenté par un mélange de carboxyméthylcellulose sodique et d'alginates de sodium, avec le rapport suivant de composants, en % en poids :
| | |
|---|---|
| Xymédon | 1,0 à 10,0 |
| Carboxyméthyl-cellulose sodique | 1,0 à 3,0 |
| Alginates de sodium | 0,2 à 0,4 |
| Glycérine | 20,0 à 30,0 |
| Stabilisant | 0,4 à 1,0 |
| Conservateur | 1,0 à 2,0 |
| Eau distillée | quantité suffisante jusqu'à 100. |

5. Composition selon l'une quelconque des revendications 1 à 4, **caractérisée en ce qu'**elle comprend en outre un nitrate d'argent comme substance active en une quantité allant de 0,1 à 0,2 % en poids.

6. Composition selon l'une quelconque des revendications 1 à 4, **caractérisée en ce qu'**elle comprend en outre du nitrate d'argent comme substances actives, 0,1 à 0,2 % en poids, et du sulfacyle sodium, 0,5 à 1,0 % en poids.

7. Composition selon l'une quelconque des revendications 1 à 4, **caractérisée en ce qu'**elle comprend en outre de la lévomycétine comme substances actives, 1,0 à 5,0 % en poids, et de l'acide succinique, 2,0 à 10,0 % en poids.

8. Procédé pour produire une composition pharmaceutique pour traiter des brûlures selon l'une quelconque des revendications 1 à 6, **caractérisé en ce que** dans un réacteur ayant une chemise d'eau dans un mélange d'eau distillée et d'un agent de rétention d'humidité, chauffé jusqu'à 50 à 60 °C, le gélifiant est dissous sous agitation pour former une masse homogène uniforme après quoi on ajoute au réacteur un conservateur et un stabilisant, avec addition d'une unité de la base ainsi obtenue à la quantité calculée d'une ou de plusieurs substances actives, préalablement réduites en poudre dans un moulin à colloïdes, et mélange de la matière afin d'obtenir la masse homogène qui est ensuite ramenée jusqu'au réacteur et mélangée avec la portion restante de la base pour former la masse homogène.

9. Procédé selon la revendication 8, **caractérisé en ce que** tout en incluant de façon additionnelle dans la composition selon l'une quelconque des revendications 1 à 4, de la lévomycétine comme substances actives en une quantité allant de 1,0 à 5,0 % en poids et de l'acide succinique en une quantité allant de 2,0 à 10,0 % en poids, elles sont dissoutes dans de l'eau chauffée à 70 °C dans un autre réacteur ayant une chemise d'eau, et la solution ainsi obtenue est ensuite combinée avec un fragment de la base contenant du xymédon.

10. Composition selon l'une quelconque des revendications 1 à 7 pour utilisation dans un procédé pour traiter des brûlures.
